# EUROPÄISCHE PATENTSCHRIFT

(11) **EP 0 710 651 B1**
(45) Veröffentlichungstag und Bekanntmachung des Hinweises auf die Patenterteilung: **17.01.2001**
(21) Anmeldenummer: 95114205.8
(22) Anmeldetag: 11.09.1995
(51) Int. Cl.: C07D 217/26, C07D 303/38, C07B 51/00, C07C 229/34

(54) **Verfahren zur Herstellung eines Decahydroisochinolino-alpha,alpha'diamino-alkohols**
Process for the preparation of a decahydroisoquinoline-alpha, alpha' diamino alcohol
Procédé pour la préparation de décahydro isoquinoléine alpha alpha'diamino alcool

(30) Priorität: 23.09.1994 CH 290494; 09.12.1994 CH 373694
(43) Veröffentlichungstag der Anmeldung: 08.05.1996
(73) Patentinhaber: F. Hoffmann-La Roche AG, 4002 Basel (CH)
(72) Erfinder: Hilpert, Hans, CH-4153 Reinach (CH)
(74) Vertreter: Witte, Hubert, Dr.

(56) Entgegenhaltungen:
- EP-A- 0 432 695
- EP-A- 0 525 880
- EP-A- 0 543 343
- EP-A- 0 635 493
- WO-A-94/11345
- TETRAHEDRON LETT., Bd. 30, Nr. 46, 1989 OXFORD, Seiten 6393-6394, SHIRAHATA,A. 'Novel and Facile Synthesis of t-butyl substituted Silanes from t-butyl Magnesium Chloride and Chlorosilanes Catalyzed by Cuprous Cyanide'
- J.ORG.CHEM., Bd. 57, 1992 Seiten 5247-5250, D'ANIELLO,F. ET AL. 'A stereoselective Method for the Preparation of HIV-1 Protease Inhibitors Based on the Lewis Acid Mediated Reaction of Allylsilanes and N-Boc-alpha-amino Aldehydes '
- J.AM.CHEM.SOC., Bd. 116, 1994 WASHINGTON, Seiten 1316-1323, KONRADI,A.W. ET AL. 'Pinacol Cross Coupling of 2-[N-(Alkoxycarbonyl) amino] Aldehydes by ....'
- ANGEW.CHEM.INT.ED.ENGL., Bd. 30, Nr. 12, Dezember 1991 WEINHEIM, Seiten 1531-1546, REETZ,M.T. 'New Approaches to the Use of Amino Acids as Chiral Building Blocks in Organic Synthesis'
- PHARMAZIE I.U.ZT. , Bd. 13, Nr. 3, 1984 WEINHEIM, Seiten 83-88, ANGRICK,M. 'Schutzgruppen : Hilfsmittel der präparativen Chemie'

## Beschreibung

Die vorliegende Erfindung betrifft ein neues Verfahren zur Herstellung eines α,α'-Diaminoalkohols, nämlich des 2-[3(S)-Amino-2(R)-hydroxy-4-phenylbutyl]-N-tert.butyl-decahydro-(4aS,8aS)-isochinolin-3(S)-carboxamids der Formel

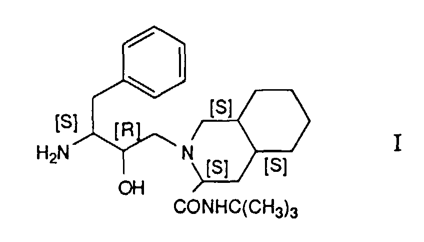

Die Verbindung der obigen Formel I ist beispielsweise in Beispiel 1 der Europäischen Patentpublikation 0.432.695 bzw. in dem U.S. Patent 5,196,438 spezifisch beschrieben und ist ein wertvolles Zwischenprodukt zur Herstellung von pharmakologisch aktiven Verbindungen. So kann die Verbindung der Formel I wie in den Beispielen 1 und 3 der genannten Publikation beschrieben in pharmakologisch aktive Verbindungen übergeführt werden, welche sich zur Behandlung viraler Infektionen eignen, insbesondere solcher Infektionen, die durch HIV und andere Retroviren verursacht werden.

Das erfindungsgemässe Verfahren ist dadurch gekennzeichnet, dass man
a) einen L-Phenylalanin-nieder-alkylester mit einem Chlorameisensäure-ester der Formel ClCOOR¹, worin R¹ nieder-Alkyl, Benzyl oder Phenyl bedeutet, umsetzt,
b) einen erhaltenen Diester der allgemeinen Formel 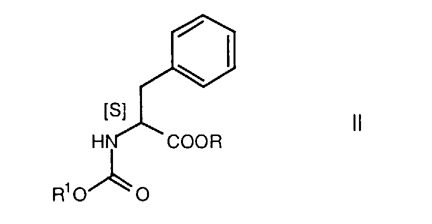 worin R nieder-Alkyl bedeutet und R¹ obige Bedeutung hat, mit halogeniertem Methyllithium umsetzt,
c) ein erhaltenes halogeniertes α-Aminoketon der allgemeinen Formel 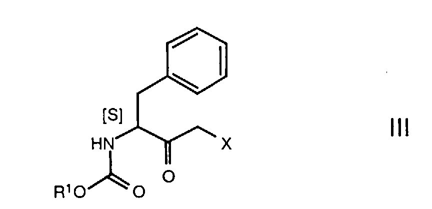 worin X Halogen bedeutet und R¹ obige Bedeutung hat, reduziert,
d) einen erhaltenen halogenierten α-Aminoalkohol der allgemeinen Formel 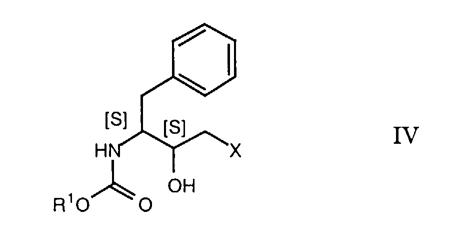 worin X und R¹ obige Bedeutung haben,
   mit einer Base cyclisiert,
e) einen erhaltenen [(S)-1-[(S)-Oxiran-2-yl]-2-phenyl-ethyl]-carbaminsäureester der allgemeinen Formel 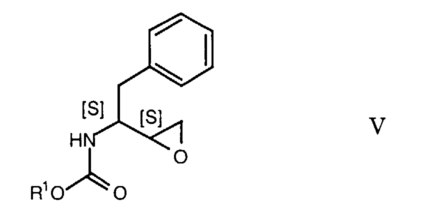 worin R¹ obige Bedeutung hat,
   mit N-tert.Butyl-decahydro-(4aS,8aS)-isochinolin-3(S)-carboxamid der Formel 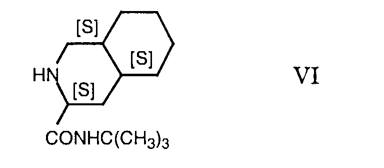 umsetzt, und
f) den entstandenen (1S,2R)-[1-Benzyl-3-[(3S,4aS,8aS)-3-tert-butoxycarbamoyl-octahydro-isochinolin-2-yl]-2-hydroxy-propyl]-carbaminsäuremethylester der Formel 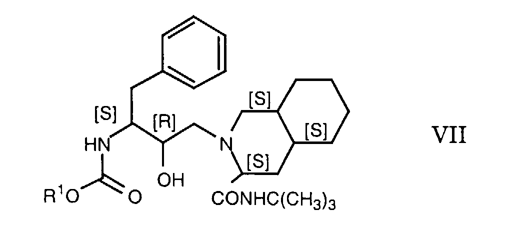 mit einer Base behandelt.

Der oben verwendete Ausdruck "nieder-Alkyl" betrifft geradkettige oder verzweigte gesättigte Kohlenwasserstoffreste mit 1 - 8, vorzugsweise 1 - 4, Kohlenstoffatomen, wie Methyl, Aethyl, n-Propyl, Isopropyl, n-Butyl, Isobutyl, sec.Butyl, tert.Butyl, Pentyl, Hexyl, Heptyl, Octyl und dergleichen. Halogen bezeichnet Brom, Chlor, Fluor oder Jod.

Im Verfahren gemäss EP 0.432.695 wird ein Halogenketon VI mit geschützter Aminogruppe mit einem Isochinolin VII zu einer aminogeschützten Ketoverbindung VIII umgesetzt, die dann zu der entsprechenden Hydroxyverbindung IX reduziert und schliesslich in die freie Aminoverbindung II überführt wird. Bei der Reduktion der Keto- zur Hydroxyverbindung wird das gewünschte 2R-OH-Stereoisomer im 1:1-Gemisch mit dem 2S-OH-Stereoisomer erhalten, was einen Verlust von 50% bezogen auf die eingesetzten Reaktionspartner bedeutet. Selbst bei quantitativer Umsetzung von VI und VII zu VIII beträgt die Ausbeute also höchstens 50%.

Im erfindungsgemässen Verfahren dagegen wird das Halogenketon mit geschützter Aminogruppe III (=VI in EP 0.432.695) zum entsprechenden Halohydrin IV mit 89% Ausbeute reduziert, wobei die Stereoselektivität überraschenderweise bei 95:5 zugunsten der erwünschten S,S-Verbindung liegt. Die anschliesende Ueberführung des Halohydrins IV in das Epoxid V erfolgt quantitativ (100%) und dessen Umsetzung mit dem Isochinolin VI (=VII in EP 0.432.695) liefert die Verbindung VII (=IX in EP 0.432.695) in 92%iger Ausbeute. Die Ausbeute an Verb. VII beim erfindungsgemässen Verfahren ist also 71.4% (ausgehend von Verb. III) gegenüber höchstens 50% beim Verfahren gemäss EP 0.432.695.

Den Diester der Formel II erhält man nach an sich bekannten Methoden durch Erwärmen von L-Phenylalanin mit Thionylchlorid in einem nieder-Alkanol, wie Methanol, eindampfen des nieder-Alkanols und Reaktion des entstandenen L-Phenylalanin-nieder-alkylesters mit einem Chlorameisensäureester ClCOOR¹, worin R¹ nieder-Alkyl, z.B. Methyl oder Aethyl, Benzyl oder Phenyl bedeutet, in einem Lösungsmittel, wie einem Keton, z.B. Methyläthylketon, oder einem Aether, z.B. tert.Butylmethyläther, oder einem Kohlenwasserstoff, vorzugsweise Toluol, in Gegenwart einer Base, wie einem nieder-Alkylamin oder einem Alkali- oder Erdalkalimetallhydroxid, vorzugsweise Kalium- oder Natriumhydroxid, in Gegenwart von Wasser, bei einer Temperatur von 0° - 60°C, vorzugsweise bei 0° - 10°C, bei pH 4 - 10, vorzugsweise 6 - 7.

Die Halomethylierung des erhaltenen Diesters II erfolgt vorzugsweise mit in situ erzeugtem halogenierten Methyllithium. Letzteres wird zweckmässigerweise mit dihalogeniertem Methan, vorzugsweise mit Brom-chlormethan, und einem nieder-Alkyl-lithium, vorzugsweise Butyl- oder Hexyllithium, in einem Aether, vorzugsweise Tetrahydrofuran, bei -20° bis -120°C, vorzugsweise -80°C, gebildet.

Die Halomethylierung des Diesters II zum halogenierten α-Aminoketon III kann zweckmässigerweise, dadurch bewerkstelligt werden, dass man
a) einen Diester der allgemeinen Formel 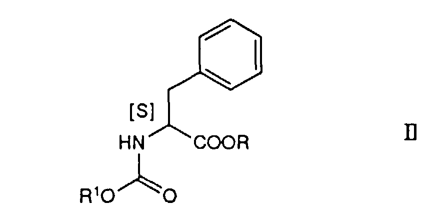 worin R nieder-Alkyl bedeutet und R¹ obige Bedeutung hat,
   mit einem nieder-Alkyl-lithium und einem Organochlorsilan der Formel ClSi(R²,R³,R⁴), worin R², R³ und R⁴ nieder-Alkyl oder Phenyl bedeuten, umsetzt und
b) eine intermediär gebildete silylgeschützte Verbindung der allgemeinen Formel VIII oder IX 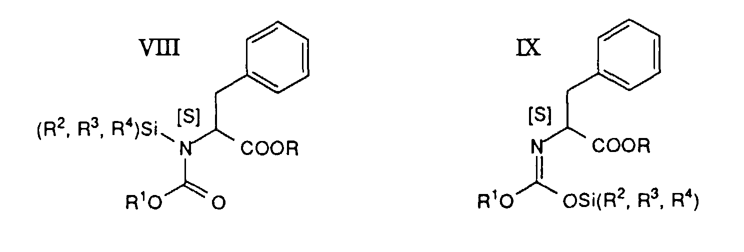 worin R, R¹, R², R³ und R⁴ obige Bedeutung haben,
   in Gegenwart von dihalogeniertem Methan und eines nieder-Alkyllithiums umsetzt.

Es wurde nämlich unerwarteter Weise gefunden, dass der vorgängige Schutz der im Diester II enthaltenen Carbamatgruppe durch eine Silylgruppe unter intermediärer Bildung der Verbindungen der obigen Formel VIII oder IX zu einer beträchtlichen Steigerung der Ausbeute führt. Als nieder-Alkyl-lithium verwendet man vorzugsweise Butyl- oder Hexyllithium und als Organochlorsilan ClSi(R²,R³,R⁴) das Chlortrimethylsilan. Ausserdem konnte ein nahezu vollständiger Umsatz mit bedeutend weniger nieder-Alkyl-lithium und dihalogeniertem Methan erzielt werden.

Die Reduktion des Halomethylketons III wird zweckmässigerweise in einem Lösungsmittel wie Toluol, Tetrahydrofuran oder einem Alkohol, vorzugsweise Methanol, Aethanol oder Isopropanol, bei einer Temperatur zwischen -30 und 80°C, vorzugsweise -15°C und 50°C, ggf. unter vermindertem Druck, mittels Natrium-bis(2-metoxyäthoxy)-aluminiumhydrid, Lithium-aluminiumhydrid, Lithium-aluminium-tri-tert.-butoxyhydrid, Natrium-borhydrid, Tetramethylammoniumborhydrid oder, vorzugsweise, mittels einem Aluminium-tri-alkoxid bzw. Lithiumaluminium-tri-alkoxihydrid durchgeführt. Der Ausdruck Alkoxide umfasst gerad- oder verzweigtkettige gesättigte Kohlenwasserstoffoxide mit 1-8, vorzugsweise 3-4, Kohlenstoffatomen, nämlich Methyl-, Aethyl-, n-Propyl-, Isopropyl-, n-Butyl-, Isobutyl-, sec.Butyl-, tert. Butyloxid sowie Pentyl-, Hexyl-, Heptyl- und Octyl-Oxide. Die Aluminiumverbindungen können identische oder verschiedene Alkoxidgruppen aufweisen. Besonders bevorzugte Verbindungen sind Aluminium-tri-isopropoxid oder Aluminium-tri-sec.-butoxid. Die Reagentien Lithium-aluminium-tri-tert.butoxyhydrid, Aluminium-tri-isopropoxid und Aluminium-tri-sec.-butoxid ergaben unerwarteterweise eine hohe Stereoselektivität von 95:5 der (1S, 2S)- und (1S, 2R)-isomeren Halohydrine IV, die aus dem Reaktionsmedium in >99% optischer Reinheit und mit hoher Ausbeute kristallisiert werden konnten.

Der Ringschluss des Halohydrins der Formel IV wird zweckmässigerweise in einem Lösungsmittel, wie Aethanol oder vorzugsweise einem Toluol/Wasser-Gemisch, in Gegenwart einer Base, wie einem Alkali- oder Erdalkalimetallhydroxid, vorzugsweise Natrium- oder Kaliumhydroxid, bei einer Temperatur zwischen 0° und 80°C, vorzugsweise 40° - 50°C, durchgeführt, wobei das gebildete Epoxid der Formel V nicht gereinigt werden muss.

Die Reaktion des Epoxides der Formel V mit dem Amid VI wird zweckmässigerweise in einem Lösungsmittel, wie einem Kohlenwasserstoff, z.B. Toluol, oder einem nieder-Alkanol, vorzugsweise Aethanol, unter Erhitzen bis zur Rückflusstemperatur, vorzugsweise bei 20° - 100°C, insbesondere bei 80°C, durchgeführt.

Die Abspaltung der N-Schutzgruppe aus der Verbindung der Formel VII erfolgt zweckmässigerweise in einem Lösungsmittel, wie Wasser, Aethanol oder einem Gemisch davon, mittels einer Base, wie Natrium- oder Kaliumhydroxid, unter Erhitzen bis zur Rückflusstemperatur, vorzugsweise bei 20° - 100°C, insbesondere bei 80°.

Die Verbindungen der Formeln III bzw. IV, worin R¹ Methyl bedeutet, insbesondere
der (S)-1-Benzyl-3-chlor-2-oxopropyl)carbaminsäuremethylester bzw.
der (1S,2S)-(1-Benzyl-3-chlor-2-hydroxypropyl)carbaminsäuremethylester,
sowie
die Verbindungen der Formeln V bzw. VII, worin R¹ Methyl bedeutet, nämlich
der [(S)-1-[(S)-Oxiran-2-yl]-2-phenethyl]carbaminsäuremethylester bzw.
der (1S,2R)-[1-Benzyl-3-[(3S,4aS,8aS)-3-tert-butoxycarbamoyl-octahydroisochinolin-2-yl]-2-hydroxypropyl]carbaminsäuremethylester,
sind neu und als solche ebenfalls Gegenstand der vorliegenden Erfindung. Die Erfindung umfasst ebenfalls die Verbindungen der Formeln I, III und IV, die durch die oben beschriebenen Verfahren erhalten werden können sowie die Verwendung der Verbindungen der allgemeinen Formeln III und IV zur Herstellung der Verbindung der Formel I.

Die als Ausgangsstoff eingesetzte Verbindung der Formel VI ist bekannt und entspricht derjenigen der Formel VII in der Europäischen Patentpublikation 0.432.695 bzw. in dem U.S. Patent 5,196,438.

Die folgenden Beispiele sollen die vorliegende Erfindung erläutern, sie jedoch in keiner Weise einschränken.

### Beispiel 1

Zu 800 ml Methanol wurden bei 0°C 160 ml Thionylchlorid getropft. Anschliessend wurde mit 330,4 g L-Phenylalanin versetzt und 2,5 Stunden auf 45°C erwärmt. Die entstandene Lösung wurde vollständig eingeengt, der Rückstand in 1,6 l Wasser aufgenommen und bei 0°C mit 185 ml Chlorameisensäuremethylester versetzt, wobei der pH-Wert mit 40% Natronlauge zwischen 6 - 7 gehalten wurde. Die Lösung wurde mit Toluol extrahiert, die Extrakte nach Waschen mit Wasser eingeengt und der Rückstand bei 45°C/0,1 mbar getrocknet, wobei man 474,6 g (100%) reinen (S)-2-Methoxycarbonylamino-3-phenyl-propionsäure-methylester erhielt. IR (KBr): 3338m (NH), 1726s br. (C=O), 1531s (Amid II).

### Beispiel 2

Zu einer Lösung von 9,50 g (S)-2-Methoxycarbonylamino-3-phenylpropionsäure-methylester und 3,22 ml Bromchlormethan in 60 ml Tetrahydrofuran wurden bei -80°C 41,7 ml einer 2,6 molaren Lösung von Hexyllithium in Hexan getropft. Anschliessend wurden weitere 2,14 ml Bromchlormethan zugegeben und erneut mit 23 ml Hexyllithiumlösung versetzt, weitere 1,54 ml Bromchlormethan zugegeben und erneut mit 7,7 ml Hexyllithiumlösung versetzt. Die Lösung wurde bei -80°C mit 15 ml 20%iger methanolischer Salzsäure versetzt, auf 22° erwärmt und mit 100 ml Wasser und 40 ml Tetrahydrofuran verdünnt. Die Phasen wurden getrennt, die organische Phase mit gesättigter Natriumchloridlösung gewaschen, getrocknet und eingeengt. Der Rückstand wurde aus 40 ml Essigester und 160 ml Hexan umkristallisiert und das Kristallisat getrocknet, wobei man 3,83 g (37%) reinen (S)-(1-Benzyl-3-chlor-2-oxopropyl)-carbaminsäure-methylester, Smp. 121° - 122°C, erhielt. IR (KBr): 3336s (NH), 1737s und 1686s (C=O), 1535s (Amid II).

### Beispiel 3

Zu einer Lösung von 9,50 g (S)-2-Methoxycarbonylamino-3-phenylpropionsäure-methylester in 60 ml Tetrahydrofuran wurden bei -80°C 15,4 ml einer 2,6 molaren Lösung von Hexyllithium in Hexan getropft. Anschliessend wurde mit 5,60 ml Chlortrimethylsilan versetzt. Die entstandene Suspension wurde gerührt und mit 3,22 ml Bromchlormethan versetzt. Anschliessend wurden 18,4 ml Hexyllithiumlösung zugesetzt. Die Lösung wurde bei -80°C mit 11 ml 20%iger methanolischer Salzsäure versetzt, auf 22° erwärmt und mit 100 ml Wasser und 40 ml Tetrahydrofuran verdünnt. Die Phasen wurden getrennt, die organische Phase mit gesättigter Natriumchloridlösung gewaschen, getrocknet und eingeengt. Der Rückstand wurde aus Essigester und Hexan umkristallisiert und das Kristallisat getrocknet, wobei man 7,20 g (70 %) reinen (S)-(1-Benzyl-3-chlor-2-oxo-propyl)-carbaminsäure-methylester, Smp. 122° - 123°C, erhielt. IR (KBr): 3336s (NH), 1737s und 1686s (C=O), 1535s (Amid II).

### Beispiel 4

A) Zu einer Suspension von 25,57 g (S)-(1-Benzyl-3-chlor-2-oxo-propyl)-carbaminsäure-methylester in 280 ml Aethanol wurden bei -15°C portionsweise 33.06 g Lithium-aluminium-tri-tert.butoxyhydrid gegeben und anschliessend mit 140 ml 3N Salzsäure und 170 ml Wasser bei O°C hydrolysiert. Die Suspension wurde auf 370 ml eingeengt, abfiltriert, der Rückstand mit Wasser/Aethanol (4:1) gewaschen und getrocknet, wobei man 23.27 g (90%) isomerenreinen (1S,2S)-(1-Benzyl-3-chlor-2-hydroxypropyl)-carbaminsäure-methylester, Smp. 163-164.5°C, erhielt. IR (KBr): 3323s, br. (NH, OH), 1689 (C=O), 1546s (Amid II).
B) Zu einer Suspension von 21.44 g Aluminium-isopropoxid in 260 ml Isopropanol wurden bei 22°C portionsweise 25.57 g (S)-(1-Benzyl-3-chlor-2-oxo-propyl)-carbaminsäure-methylester gegeben. Die Suspension wurde bei 50°/400 mbar 2 Stunden gerührt, mit 100 ml 3N Salzsäure bei 0° hydrolysiert, anschliessend auf ein Volumen von 125 ml eingeengt, mit 125 ml Wasser verdünnt, auf 0° gekühlt und abfiltriert. Der Rückstand wurde mit Wasser/Isopropanol (4:1) gewaschen und getrocknet, wobei man 23.01 g (89%) isomerenreinen (1S,2S)-(1-Benzyl-3-chlor-2-hydroxypropyl)-carbaminsäure-methylester, Smp. 162-163.5°C, erhielt. IR (KBr): 3323s, br. (NH, OH), 1689 (C=O), 1546s (Amid II).

### Beispiel 5

Zu einer Suspension von 46,03 g (S)-(1-Benzyl-3-chlor-2-oxo-propyl)-carbaminsäure-methylester in 275 ml Methanol wurden bei -15°C portionenweise 3,75 g Natriumborhydrid gegeben. Es wurde 1,5 Stunden gerührt, mit 21 ml Essigsäure und 460 ml Wasser verdünnt, 1 Stunde bei -15°C gerührt und abfiltriert. Der Rückstand wurde mit Wasser gewaschen, aus 430 ml Isopropanol umkristallisiert und das Kristallisat getrocknet, wobei man 28,68 g (62%) eines 98 : 2-Gemisches der (1S,2S): (1S,2R)-Isomeren des (1-Benzyl-3-chlor-2-hydroxy-propyl)-carbaminsäure-methylesters, Smp. 161,5° - 162,5°C, erhielt. IR (KBr): 3323s, br. (NH, OH), 1689s (C=O), 1546s (Amid II).

### Beispiel 6

Ein Gemisch von 28.35 g(1S,2S)-(1-Benzyl-3-chlor-2-hydroxy-propyl)-carbaminsäuremethylester, 8,8 g Natriumhydroxid, 110 ml Toluol und 110 ml Wasser wurde bei 40°C gerührt, die organische Phase dann mit Wasser gewaschen und eingeengt. Der zurückbleibende [(S)-1-[(S)-Oxiran-2-yl]-2-phenyl-ethyl]-carbaminsäure-methylester wurde in 130 ml Aethanol aufgenommen, mit 26,22 g N-tert.Butyl-decahydro-(4aS,8aS)-isochinolin-3(S)-carboxamid versetzt und 5,3 Stunden am Rückfluss erhitzt. Die Suspension wurde mit 80 ml Wasser verdünnt, auf 22° abgekühlt und abfiltriert, der Rückstand mit einem 1:1-Gemisch von Aethanol/Wasser gewaschen und getrocknet, wobei man 46,30 g (92%) des (1S,2R)-[1-Benzyl-3-[(3S,4aS,8aS)-3-tert-butoxy-carbamoyl-octahydroisochinolin-2-yl]-2-hydroxy-propyl]-carbaminsäure-methylesters, Smp. 194° - 195°C, erhielt. IR (KBr): 3411m und 3317m (NH, OH), 1715s und 1659s (C=O), 1548s (Amid II).

### Beispiel 7

Eine Suspension von 41,37 g (1S,2R)-[1-Benzyl-3-[(3S,4aS,8aS)-3-tert-butoxycarbamoyl-octahydro-isochinolin-2-yl]-2-hydroxy-propyl]-carbaminsäure-methylester und 23,0 g Natriumhydroxid in 90 ml Aethanol und 90 ml Wasser wurde 3,5 Stunden am Rückfluss erhitzt, mit 45 ml Wasser verdünnt und auf 22° abgekühlt, der ausgefallene Festkörper abfiltriert, mit einem 1:4-Gemisch von Aethanol/Wasser gewaschen und getrocknet, wobei man 35,35 g (98%) reines 2-[3(S)-Amino-2(R)-hydroxy-4-phenylbutyl]-N-tert.butyl-decahydro-(4aS,8aS)-isochinolin-3(S)-carboxamid, Smp. 173° - 175°C, erhielt. IR (KBr): 3435m, br. (NH, OH), 1665s (C=O), 1562m (Amid II).

## Patentansprüche

1. Verfahren zur Herstellung von 2-[3(S)-Amino-2(R)-hydroxy-4-phenyl-butyl]-N-tert.butyl-decahydro-(4aS,8aS)-isochinolin-3(S)-carboxamid der Formel 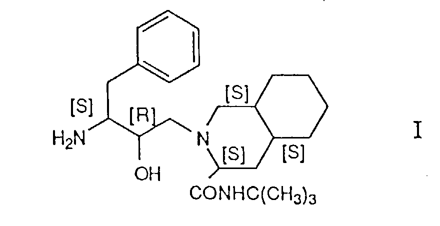 dadurch gekennzeichnet, dass man
a) einen L-Phenylalanin-nieder-alkylester mit einem Chlorameisensäure-ester der Formel ClCOOR¹, worin R¹ nieder-Alkyl, Benzyl oder Phenyl bedeutet, umsetzt,
b) einen erhaltenen Diester der allgemeinen Formel 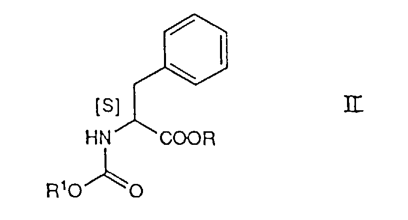 worin R nieder-Alkyl bedeutet und R¹ obige Bedeutung hat,
mit halogeniertem Methyllithium umsetzt,
c) ein erhaltenes halogeniertes α-Aminoketon der allgemeinen Formel 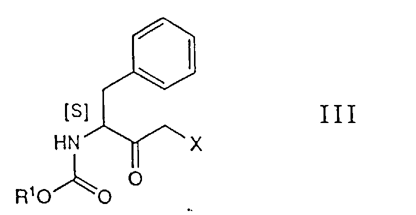 worin X Halogen bedeutet und R¹ obige Bedeutung hat,
reduziert,
d) einen erhaltenen halogenierten α-Aminoalkohol der allgemeinen Formel 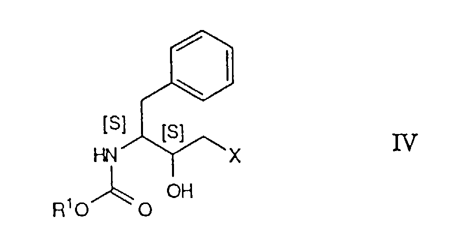 worin X und R¹ obige Bedeutung haben,
mit einer Base cyclisiert,
e) einen erhaltenen [(S)-1-[(S)-Oxiran-2-yl]-2-phenyl-ethyl]-carbaminsäureester der allgemeinen Formel 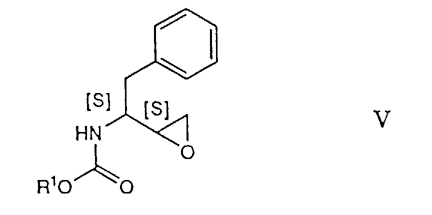 worin R¹ obige Bedeutung hat,
mit N-tert.Butyl-decahydro-(4aS,8aS)-isochinolin-3(S)-carboxamid der Formel 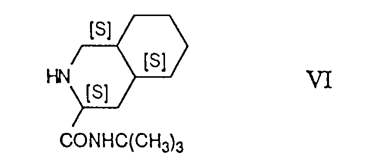 umsetzt, und
f) den entstandenen (1S,2R)-[1-Benzyl-3-[(3S,4aS,8aS)-3-tert-butoxycarbamoyl-octahydro-isochinolin-2-yl]-2-hydroxy-propyl]-carbaminsäuremethylester der Formel 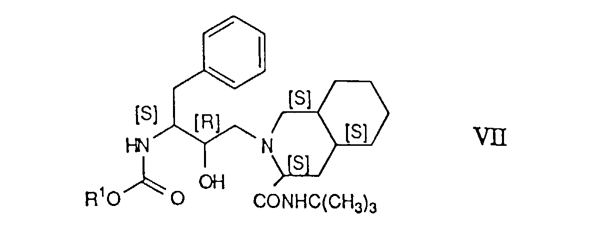 mit einer Base behandelt.

2. Verfahren zur Reduktion eines α-Aminoketons der allgemeinen Formel 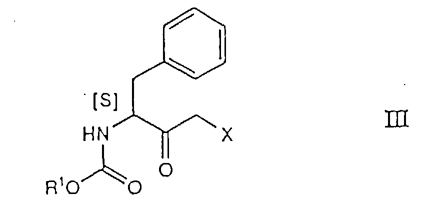 worin X Halogen und R¹ nieder-Alkyl, Benzyl oder Phenyl bedeuten, worin als Reduktionsmittel Aluminiumtrialkoxide bzw. Lithiumaluminium-trialkoxyhydride verwendet werden.

3. Die Verbindungen der Formeln III bzw. IV, worin R¹ Methyl bedeutet, insbesondere
der (S)-1-Benzyl-3-chlor-2-oxopropyl)carbaminsäuremethylester bzw.
der (1S,2S)-(1-Benzyl-3-chlor-2-hydroxypropyl)carbaminsäuremethylester.

4. Die Verbindungen der Formeln V bzw. VII, worin R¹ Methyl bedeutet, nämlich
der [(S)-1-[(S)-Oxiran-2-yl]-2-phenethyl]carbaminsäuremethylester bzw.
der (1S,2R)-[1-Benzyl-3-[(3S,4aS,8aS)-3-tert-butoxycarbamoyl-octahydroisochinolin-2-yl]-2-hydroxypropyl]carbaminsäuremethylester.

5. Verwendung von Verbindungen der Formeln III bzw. IV, worin R¹ Methyl bedeutet, und/oder von Verbindungen der Formeln V bzw. VII, worin R¹ Methyl bedeutet zur Herstellung von 2-[3(S)-Amino-2(R)-hydroxy-4-phenyl-butyl]-N-tert.butyl-decahydro-(4aS,8aS)-isochinolin-3(S)-carboxamids der Formel

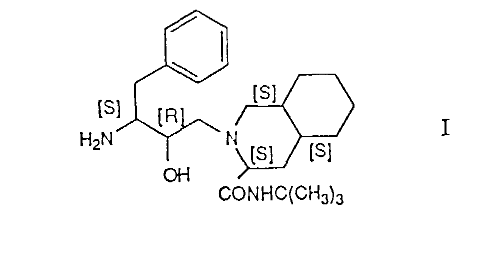

6. Verwendung von (S)-1-Benzyl-3-chlor-2-oxopropyl)carbaminsäuremethylester, (1S,2S)-(1-Benzyl-3-chlor-2-hydroxypropyl)carbaminsäuremethylester, [(S)-1-[(S)-Oxiran-2-yl]-2-phenethyl]carbaminsäuremethylester und/oder (1S,2R)-[1-Benzyl-3-[(3S,4aS,8aS)-3-tert-butoxycarbamoyl-octahydroisochinolin-2-yl]-2-hydroxypropyl]-carbaminsäure-methylester zur Herstellung von 2-[3(S)-Amino-2(R)-hydroxy-4-phenyl-butyl]-N-tert.butyl-decahydro-(4aS,8aS)-isochinolin-3(S)-carboxamids der Formel

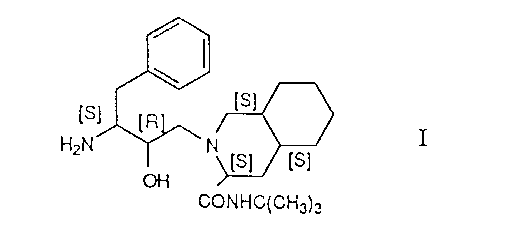

## Claims

1. A process for the manufacture of 2-[3(S)-amino-2(R)-hydroxy-4-phenylbutyl]-N-tert.butyl-decahydro-(4aS,8aS)-isoquinoline-3(S)-carboxamide of the formula 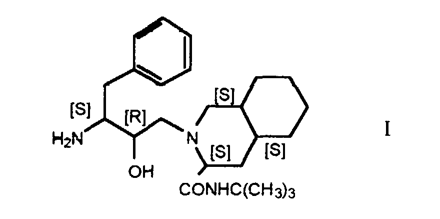 characterized by
a) reacting a L-phenylalanine lower-alkyl ester with a chloroformic acid ester of the formula ClCOOR¹, wherein R¹ signifies lower-alkyl, benzyl or phenyl,
b) reacting a resulting diester of the general formula 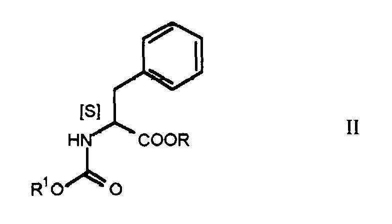 wherein R signifies lower-alkyl and R¹ has the above significance,
with halogenated methyllithium,
c) reducing a resulting halogenated α-aminoketone of the general formula 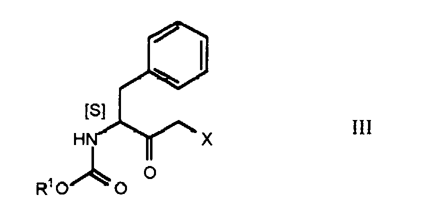 wherein X signifies halogen and R¹ has the above significance,
d) cyclizing a resulting halogenated α-aminoalcohol of the general formula 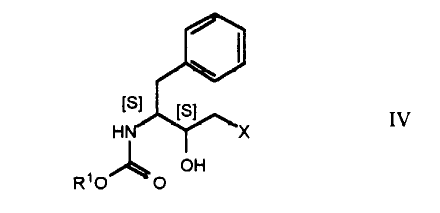 wherein X and R¹ have the above significance,
with a base,
e) reacting a resulting [(S)-1-[(S)-oxiran-2-yl]-2-phenyl-ethyl]-carbamic acid ester of the general formula 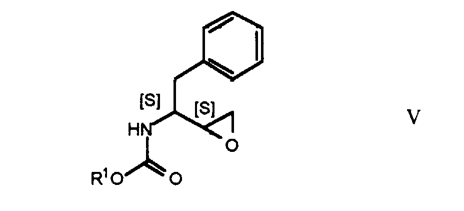 wherein R¹ has the above significance,
with N-tert.butyl-decahydro-(4aS,8aS)-isoquinoline-3(S)-carboxamide of the formula 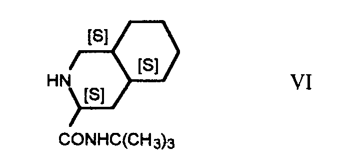 and
f) treating the resulting methyl (1S,2R)-[1-benzyl-3-[(3S,4aS,8aS)-3-tert-butoxycarbamoyl-octahydro-isoquinolin-2-yl]-2-hydroxy-propyl]-carbamate of the formula 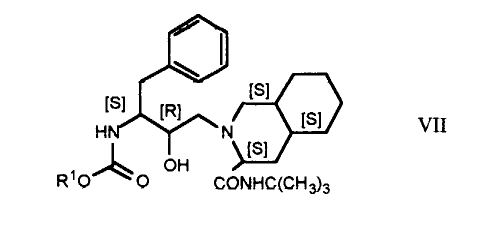 with a base.

2. A method for the reduction of an α-aminoketone of the general formula 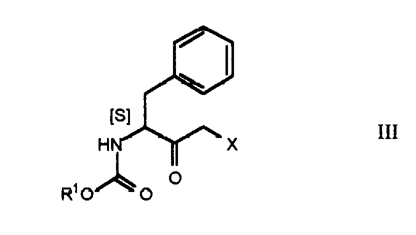 wherein X signifies halogen and R¹ signifies lower-alkyl, benzyl or phenyl, wherein an aluminium trialkoxide or lithium aluminium trialkoxy hydride is used as the reducing agent.

3. The compounds of formulae III and, respectively, IV in which R¹ signifies methyl, especially
methyl (S)-1-benzyl-3-chloro-2-oxopropyl)carbamate and, respectively,
methyl (1S,2S)-(1-benzyl-3-chloro-2-hydroxypropyl)carbamate.

4. The compounds of formulae V and, respectively, VII in which R¹ signifies methyl, namely
methyl [(S)-1-[(S)-oxiran-2-yl]-2-phenethyl]carbamate and, respectively,
methyl (1S,2R)-[1-benzyl-3-[(3S,4aS,8aS)-3-tert-butoxycarbamoyl-octahydro-isoquinolin-2-yl]-2-hydroxypropyl]carbamate.

5. The use of compounds of formulae III and, respectively, IV in which R¹ signifies methyl and/or of compounds of formulae V and, respectively, VII in which R¹ signifies methyl for the manufacture of 2-[3(S)-amino-2(R)-hydroxy-4-phenyl-butyl)-N-tert.butyl-decahydro-(4aS,8aS)-isoquinoline-3(S)-carboxamide of the formula

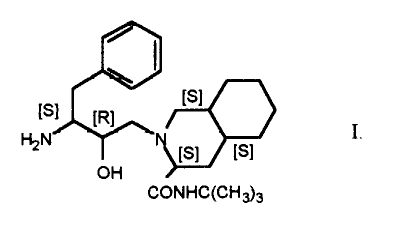

6. The use of methyl (S)-1-benzyl-3-chloro-2-oxopropyl)carbamate, methyl (1S,2S)-(1-benzyl-3-chloro-2-hydroxypropyl)carbamate, methyl [(S)-1-[(S)-oxiran-2-yl]-2-phenethyl]carbamate and/or methyl (1S,2R)-[1-benzyl-3-[(3S,4aS,8aS)-3-tert.butoxycarbamoyl-octahydroisoquinolin-2-yl]-2-hydroxypropyl]carbamate for the manufacture of 2-[3(S)-amino-2(R)-hydroxy-4-phenyl-butyl]-N-tert.butyl-decahydro-(4aS,8aS)-isoquinoline-3(S)-carboxamide of the formula

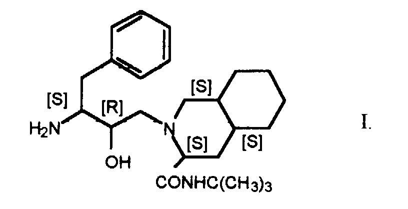

## Revendications

1. Procédé de préparation de 2-[3(S)-amino-2(R)-hydroxy-4-phénylbutyl]-N-tert.-butyldécahydro-(4aS,8aS)-isoquinoléine-3(S)-carboxamide de formule : 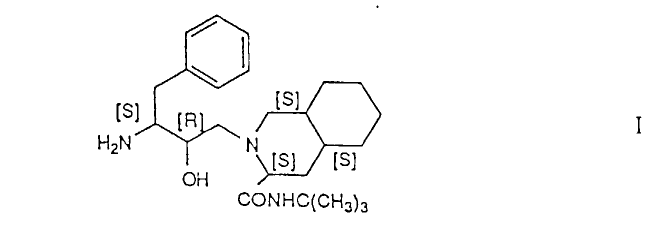 caractérisé en ce que
on fait réagir un ester alkylique inférieur de L-phénylalanine avec un ester d'acide chloroformique de formule CICOOR¹, dans laquelle R¹ représente un alkyle inférieur, un benzyle ou un phényle,
on fait réagir un diester obtenu de formule générale : 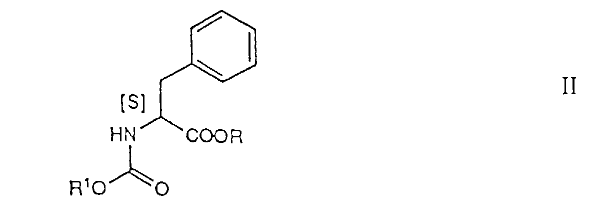
dans laquelle R représente un alkyle inférieur et R¹ a la signification donnée ci-dessus, avec du méthyllithium halogéné,
on réduit une α-aminocétone halogénée obtenue de formule générale : 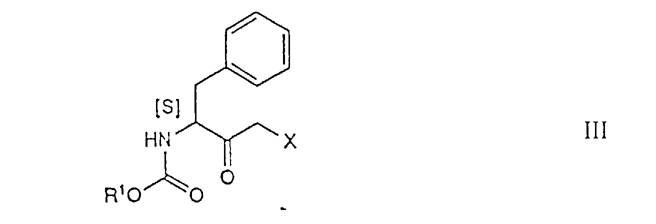
dans laquelle X représente un halogène et R¹ a la signification donnée ci-dessus,
on cyclise avec une base un α-aminoalcool halogéné obtenu de formule générale : 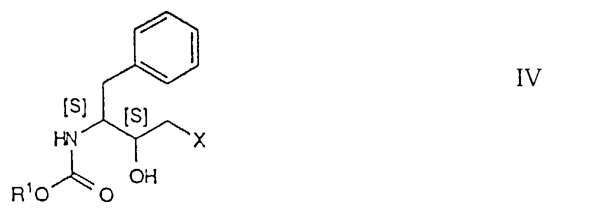
dans laquelle X et R¹ ont la signification donnée ci-dessus, et
on fait réagir un ester d'acide [(S)-1-[(S)-oxiran-2-yl]-2-phényléthyl]-carbamique de formule générale : 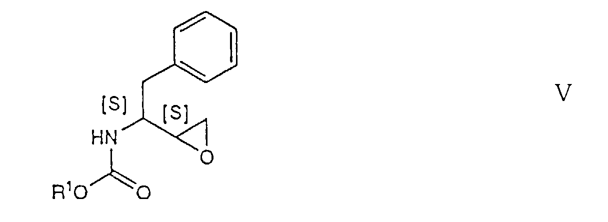
dans laquelle R¹ a la signification donnée ci-dessus avec le N-tert.-butyldécahydro-(4aS,8aS)-isoquinoléine-3(S)-carboxamide de formule : 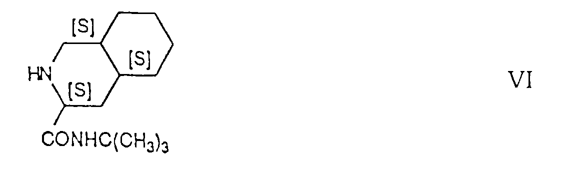 et
on traite avec une base l'ester méthylique de l'acide (1S,2R)-[1-benzyl-3-[(3S,4aS,8aS)-3-tert.-butoxycarbamoyloctahydroisoquinoléin-2-yl]-2-hydroxypropyl]-carbamique de formule :

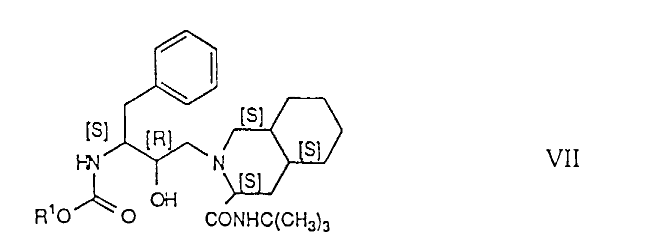

2. Procédé de réduction d'une α-aminocétone de formule générale : 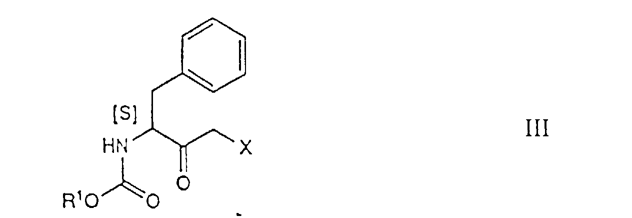 dans laquelle X représente un halogène et R¹ un alkyle inférieur, un benzyle ou un phényle, où l'on utilise comme réducteurs des trialcoxydes d'aluminium ou selon les cas des trialcoxy hydrures de lithium-aluminium.

3. Les composés de formules III ou selon les cas IV dans lesquelles R¹ représente un méthyle, en particulier l'ester méthylique de l'acide (S)-1-benzyl-3-chloro-2-oxopropyl)-carbamique ou selon les cas l'ester méthylique de l'acide (1S,2S)-(1-benzyl-3-chloro-2-hydroxypropyl)-carbamique.

4. Les composés de formules V ou selon les cas VII, dans lesquelles R¹ représente un méthyle, à savoir l'ester méthylique de l'acide [(S)-1-[(S)-oxiran-2-yl]-2-phényléthyl]-carbamique ou selon les cas l'ester méthylique de l'acide (1S,2R)-[(1-benzyl-3-[(3S,4aS,8aS)-3-tert.-butoxycarbamoyloctahydroisoquinoléin-2-yle]-2-hydroxypropyl]-carbamique.

5. Utilisation de composés de formules III ou selon les cas IV, dans lesquelles R¹ représente un méthyle, et/ou de composés de formules V ou selon les cas VII, dans lesquelles R¹ représente un méthyle, pour la préparation de 2-[3(S)-amino-2(R)-hydroxy-4-phénylbutyl]-N-tert.butyldécahydro-(4aS,8aS)-isoquinoléine-3(S)-carboxamide de formule :

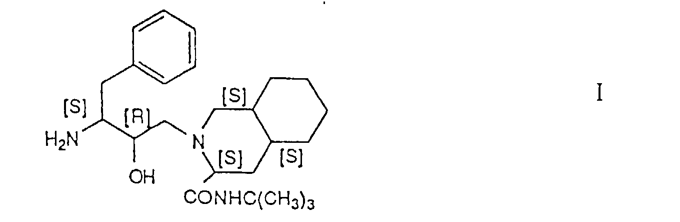

6. Utilisation de d'ester méthylique de l'acide (S)-1-benzyl-3-chloro-2-oxopropyl)carbamique, d'ester méthylique de l'acide (1S,2S)-1-benzyl-3-chloro-2-hydroxypropyl)carbamique, d'ester méthylique de l'acide [(S)-1-[(S)-oxiran-2-yl]-2-phénéthyl]carbamique et/ou d'ester méthylique de l'acide (1S,2R)-[1-benzyl-3-[(3S,4aS,8aS)-3-tert.-butoxycarbamoyloctahydroisoquinoléin-2-yl]-2-hydroxypropyl]-carbamique pour la préparation du 2-[3(S)-amino-2(R)-hydroxy-4-phénylbutyl]-N-tert.-butyldécahydro-(4aS,8aS)-isoquinoléine-3(S)-carboxamide de formule :
